# EUROPEAN PATENT APPLICATION

(11) **EP 4 491 615 A1**
(43) Date of publication of application: **15.01.2025**
(21) Application number: 24187733.1
(22) Date of filing: 10.07.2024
(51) Int. Cl.: C07D 207/12, A61K 9/00, A61K 47/00

(54) **LIPIDS FOR ACTIVE AGENT DELIVERY**

(30) Priority: 10.07.2023 US 202363512852 P
(71) Applicant: Taiwan Bio-Manufacturing Corporation, Taipei City 114063 (TW)
(72) Inventor: PENG, Shao-Zheng, 11571 TAIPEI CITY (TW); HUANG, JIAO-REN, 11571 TAIPEI CITY (TW); LAM, KING, 11571 TAIPEI CITY (TW); LU, YANN-YU, 11571 TAIPEI CITY (TW)
(74) Representative: 2K Patentanwälte Blasberg Kewitz & Reichel

(57) **Abstract**

The present disclosure provides an ionizable amine-containing lipid. Particularly, the ionizable amine-containing lipid is able to deliver a nucleic acid.

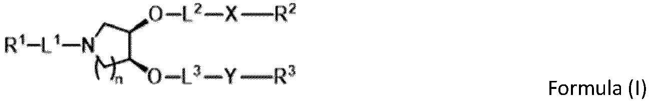

## Description

### PRIORITY INFORMATION

The subject application claims priority to and benefit of U.S. Provisional Patent Application No. 63/512,852, filed July 10, 2023, the content of which is incorporated herein by reference in its entirety.

### SEQUENCE LISTING

The instant application contains a Sequence Listing which is submitted electronically in .xml format and is hereby incorporated by reference in its entirety. The .xml copy, created on July 4, 2024, is named "EP0642_SeqListing.xml" and is 4 kilobytes in size.

### FIELD OF THE DISCLOSURE

The present disclosure relates to lipids, and the use of such lipids for active agent delivery.

### DESCRIPTION OF THE RELATED ART

The utilization of lipid nanoparticles (LNPs) that consist of ionizable amine-containing lipids has emerged as a promising strategy for delivering biologically active agents, particularly nucleotides such as antisense oligonucleotide (ASO), siRNA, miRNA, mRNAs, and guide RNAs (gRNAs), into cells. These LNPs, which incorporate ionizable amine-containing lipids, have demonstrated their essential role in facilitating the effective transportation of oligonucleotide agents across cell membranes. Additionally, they offer a valuable approach for introducing gene editing components and compositions into living cells, thereby propelling the field of genetic manipulation forward.

Achieving successful cellular uptake of biologically active agents, such as proteins, nucleic acid-based drugs, and their derivatives, presents considerable challenges. This difficulty is particularly pronounced for drugs containing sizable oligonucleotides like mRNA.

### SUMMARY OF THE INVENTION

The present disclosure provides an ionizable amine-containing lipid/composition that enhances the delivery of promising gene editing technologies into cells, for example, the delivery of CRISPR/Cas9 system components. One illustrative ionizable amine-containing lipid/composition involves the incorporation of mRNA encoding a nuclease, coupled with its corresponding guide RNA, as a means of achieving effective delivery.

The present disclosure demonstrates the utilization of the ionizable amine-containing lipid and their salts (including pharmaceutically acceptable salts) for effectively delivering biologically active agents. These ionizable amine-containing lipids and salts are valuable for delivering such active agents into cells, facilitating the creation of engineered cells. Additionally, the disclosed ionizable amine-containing lipids have demonstrated their usefulness as lipids with ionizable properties in the development of compositions based on lipid nanoparticles.

In one aspect, the present disclosure provides a lipid of Formula (I), or pharmaceutically acceptable salts, solvates, hydrates, polymorphs, tautomers, stereoisomers, or isotopically enriched derivatives thereof, 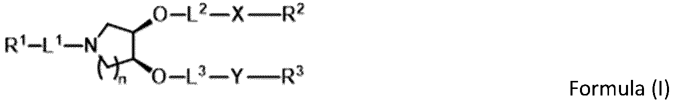 wherein:
n is 1 or 2;
R¹ is amino, monoalkylamino, dialkylamino, hydroxy, alkoxy, carbamoyl, monoalkylcarbamoyl, dialkylcarbamoyl, pyrrolidinyl, piperidyl or morpholinyl;
R² and R³ are each independently an alkyl, alkenyl or alkynyl having 5 to 30 carbon atoms; in some embodiments, R² and R³ are the same or different; in another embodiments, the alkyl, alkenyl or alkynyl may be linear or banched;
L¹ is an alkyl having 2 to 6 carbon atoms;
L² and L³ are each independently an alkyl having 2 to 9 carbon atoms; and
X and Y are each independently -C(O)-O- or -O-C(O)-.

In some embodiments,
n is 1;
R¹ is hydroxy;
R² and R³ are each independently -CH((CH₂)ₚCH₃)((CH₂)_{q}CH₃) or -(CH₂)ₘCH((CH₂)ₚCH₃)((CH₂)_{q}CH₃), wherein m is an integer from 1 to 3, and p and q are each independently an integer from 1 to 12;
L¹ is an alkyl having 2 to 6 carbon atoms;
L² and L³ are each independently an alkyl having 2 to 6 carbons atoms; and
X and Y are each independently -C(O)-O- or -O-C(O)-.

In some embodiments,
n is 1;
R¹ is hydroxy;
R² and R³ are each independently -CH((CH₂)ₚCH₃)((CH₂)_{q}CH₃) or -CH₂CH((CH₂)ₚCH₃)((CH₂)_{q}CH₃), wherein p and q are each independently an integer from 3 to 9;
L¹ is an alkyl having 2 to 6 carbon atoms;
L² and L³ are each independently an alkyl with 2 to 6 carbons atoms; and
X and Y are each independently -C(O)-O- or -O-C(O)-.

Examples of the lipid include, but are not limited to di(heptadecan-9-yl)3,3'-(((3R,4S)-1-(2-hydroxyethyl)pyrrolidine-3,4-diyl)bis(oxy))dipropionate, di(heptadecan-9-yl)3,3'-((3R,4S)-1-(3-hydroxypropyl)pyrrolidine-3,4-diyl)bis(oxy))propionate, di(heptadecan-9-yl)5,5'-(((3S,4R)-1-(3-hydroxypropyl)pyrrolidine-3,4-diyl)bis(oxy))dipentanoate, di(heptadecan-9-yl)5,5'-(((3S,4R)-1-(2-hydroxyethyl)pyrrolidine-3,4-diyl)bis(oxy))dipentanoate, (((3S,4R)-1-(3-hydroxypropyl)pyrrolidine-3,4-diyl)bis(oxy))bis(pentane-5,1-diyl)bis(2-hexyldecanoate), (((3S,4R)-1-(2-hydroxyethyl)pyrrolidine-3,4-diyl)bis(oxy))bis(pentane-5,1-diyl)bis(2-hexyldecanoate), di(heptadecan-9-yl)3,3'-(((3R,4S)-1-(4-hydroxybutyl)pyrrolidine-3,4-diyl)bis(oxy))dipropionate, (((3R,4S)-1-(3-hydroxypropyl)pyrrolidine-3,4-diyl)bis(oxy))bis(butane-4,1-diyl)bis(2-hexyldecanoate), (((3S,4R)-1-(5-hydroxypentyl)pyrrolidine-3,4-diyl)bis(oxy))bis(pentane-5,1-diyl)bis(2-hexyldecanoate), (((3S,4R)-1-(6-hydroxyhexyl)pyrrolidine-3,4-diyl)bis(oxy))bis(pentane-5,1-diyl)bis(2-hexyldecanoate), di(heptadecan-9-yl)6,6'-(((3R,4S)-1-(3-hydroxypropyl)pyrrolidine-3,4-diyl)bis(oxy))dihexanoate, di(heptadecan-9-yl)6,6'-(((3R,4S)-1-(5-hydroxypentyl)pyrrolidine-3,4-diyl)bis(oxy))dihexanoate, di(heptadecan-9-yl)6,6'-(((3R,4S)-1-(4-hydroxybutyl)pyrrolidine-3,4-diyl)bis(oxy))dihexanoate, (((3S,4R)-1-(3-hydroxypropyl)pyrrolidine-3,4-diyl)bis(oxy))bis(hexane-6,1-diyl)bis(hexyldecanoate), (((3S,4R)-1-(5-hydroxypentyl)pyrrolidine-3,4-diyl)bis(oxy))bis(hexane-6,1-diyl)bis(2-hexyldecanoate), (((3S,4R)-1-(4-hydroxybutyl)pyrrolidine-3,4-diyl)bis(oxy))bis(hexane-6,1-diyl)bis(2-hexyldecanoate), bis(2-octyldodecyl)5,5'-(((3R,4S)-1-(3-hydroxypropyl)pyrrolidine-3,4-diyl)bis(oxy))dipentanoate, bis(2-octyldodecyl)5,5'-(((3R,4S)-1-(5-hydroxypentyl)pyrrolidine-3,4-diyl)bis(oxy))dipentanoate, bis(2-octyldodecyl)5,5'-(((3R,4S)-1-(6-hydroxyhexyl)pyrrolidine-3,4-diyl)bis(oxy))dipentanoate, bis(2-butyloctyl)5,5'-(((3R,4S)-1-(3-hydroxypropyl)pyrrolidine-3,4-diyl)bis(oxy))dipentanoate, bis(2-butyloctyl)5,5'-(((3R,4S)-1-(5-hydroxypentyl)pyrrolidine-3,4-diyl)bis(oxy))dipentanoate, and bis(2-butyloctyl)5,5'-(((3R,4S)-1-(6-hydroxyhexyl)pyrrolidine-3,4-diyl)bis(oxy))dipentanoate.

In some embodiments, the pKₐ of a protonated form of the lipid is about 5.0 to about 9.0.

In another aspect, the present disclosure provides an active agent delivery vehicle comprising the lipid or pharmaceutically acceptable salts, solvates, hydrates, polymorphs, tautomers, stereoisomers, or isotopically enriched derivatives thereof as disclosed herein.

Examples of the active agent delivery vehicle include, but are not limited to, liposomes, single emulsions, micelles, nanoemulsions, lipid nanoparticles (LNPs), nanostructured lipid carriers (NLC), and self-emulsifying drug delivery system (SEDDS).

In some embodiments of the disclosure, the active agent delivery vehicle further comprises one or more lipids. Examples of the lipids include, but are not limited to, neutral lipids, steroid lipids, and PEGylated lipids.

Examples of the neutral lipids include, but are not limited to, 1,2-dilinoleoyl-sn-glycero-3-phosphocholines (DLPC), 1,2-dimyristoleoyl-sn-glycero-3-phosphocholines (DMPC), 1,2-dioleoyl-sn-glycero-3-phosphocholines (DOPC), 1,2-dipalmitoyl-sn-glycero-3-phosphocholines (DPPC), 1,2-distearoyl-sn-glycero-3-phosphatidylcholines (DSPC), 1,2-diundecanoyl-sn-glycero-3-phosphatidylcholines (DUPC), 1-plamitoyl-2-oleoyl-sn-glycero-3-phosphocholines (POPC), 1,2-di-O-octadecenyl-sn-glycero-3-phosphatidylcholines (18:0 Diether PC), 1-oleoyl-2-cholesterylhemisuccinyl-sn-glycero-3-phosphocholines (OChemsPC), 1-O-hexadecyl-sn-glycero-3-phosphatidylcholines (C16 Lyso PC), 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines (DOPE), 1,2-diphytanyl-sn-glycero-3-phosphoethanolamines (ME 16.0 PE), 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3-phosphoethanolamines, 1,2-dioleoyl-sn-glycero-3-phospho-rac-(1-glycerol) sodium salts (DOPG), dioleoyl phosphatidylserines (DOPS), dipalmitoylphosphatidylglycerols (DPPG), palmitoyloleoyl phosphatidylethanolamines (POPE), distearoyl phosphatidylethanolamines (DSPE), dipalmitoyl phosphatidylethanolamines (DPPE), dimyristoleoyl phosphoethanolamines (DMPE), 1-stearoyl-2-oleoyl-stearoylethanolamines (SOPE), 1-stearoyl-2-oleoyl-phosphatidylcholines (SOPC), sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, and lysophosphatidylethanolamines (LPE), and combinations thereof.

Examples of the steroid lipids include, but are not limited to, cholesterols, coprostanols, sitosterols, ergosterols, campesterols, stigmasterols, brassicasterol tomatidines, ursolic acids, and α-tocopherols, and combinations thereof.

Examples of the PEGylated lipids include, but are not limited to, 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), and PEG-dialkyloxypropyl (PEG-DAA), and combinations thereof.

In another aspect, the present disclosure provides a composition comprising the active agent delivery vehicle as disclosed herein and an active agent. In some embodiments of the disclosure, the composition is a pharmaceutical composition.

In some embodiments of the disclosure, the active agent is encapsulated in the active agent delivery vehicle.

In some embodiments of the disclosure, the active agent includes a nucleic acid. Examples of the nucleic acid include, but are not limited to, antisense oligonucleotide, plasmid, interfering nucleic acid, aptamer, microRNA (miRNA), Dicer-substrate RNA (dsRNA), small hairpin RNA (shRNA), mRNA (messenger RNA), guide RNA (gRNA), miRNA inhibitor (antagomir), and ribozyme, and combinations thereof.

In some embodiments of the disclosure, the mRNA encodes an enzyme, an antigen, an antibody or an antigen binding fragment.

In some embodiments of the disclosure, the composition further comprises one or more pharmaceutically acceptable excipients or adjuvants.

In another aspect, the present disclosure provides a method for treating or preventing a disease or disorder, comprising administering the composition as disclosed herein to a subject in need thereof.

In another aspect, the present disclosure provides a method for introducing a nucleic acid into a target cell or transfecting the target cell with the nucleic acid, comprising bringing the composition as disclosed herein into contact with the target cell.

In another aspect, the present disclosure provides a method of modulating the expression of a target gene in a cell, comprising providing to the cell the composition as disclosed herein.

The present invention is described in detail in the following sections. Other characteristics, purposes and advantages of the present invention can be found in the detailed description and claims.

### DETAILED DESCRIPTION

The present disclosure can be more readily understood by reference to the following detailed description of various embodiments of the disclosure, the examples, and the chemical drawings and tables with their relevant descriptions. It is to be understood that unless otherwise specifically indicated by the claims, the disclosure is not limited to specific preparation methods, carriers or formulations, or to particular modes of formulating the extract of the disclosure into products or compositions intended for topical, oral or parenteral administration, because as one of ordinary skill in the relevant arts is well aware, such factors can, of course, vary. It is also to be understood that the terminology used herein is for the purpose of describing particular embodiments only and is not intended to be limiting.

As utilized in accordance with the present disclosure, the following terms, unless otherwise indicated, shall be understood to have the following meaning:
Often, ranges are expressed herein as from "about" one particular value and/or to "about" another particular value. When such a range is expressed, an embodiment includes the range from the one particular value and/or to the other particular value. Similarly, when values are expressed as approximations, by use of the word "about," it will be understood that the particular value forms another embodiment. It will be further understood that the endpoints of each of the ranges are significant both in relation to and independently of each other.

"Optional" or "optionally" means that the subsequently described event or circumstance may or may not occur, and that the description includes instances where said event or circumstance occurs and instances where it does not. For example, the phrase "optionally comprising an agent" means that the agent may or may not exist.

It must be noted that, as used in the specification and the appended claims, the singular forms "a," "an" and "the" include plural referents unless the context clearly dictates otherwise. Thus, unless otherwise required by context, singular terms shall include the plural and plural terms shall include the singular.

The term "subject" as used herein denotes any animal, preferably a mammal, and more preferably a human. The examples of subjects include humans, non-human primates, rodents, guinea pigs, rabbits, sheep, pigs, goats, cows, horses, dogs and cats.

The term "treating" or "treatment" as used herein denotes reversing, alleviating, inhibiting the progress of, or improving the disorder, disease or condition to which such term applies, or one or more symptoms of such disorder, disease or condition.

The term "preventing" or "prevention" is recognized in the art, and when used in relation to a condition, it includes administering, prior to onset of the condition, an agent to reduce the frequency or severity of or delay the onset of symptoms of a medical condition in a subject relative to a subject which does not receive the agent.

The term "pharmaceutical composition" refers to a drug and/or vaccine for treating and/or preventing one or more diseases or disorders in a subject in need thereof. The pharmaceutical compositions can act systematically or locally. For this purpose, they can be administered by appropriate routes such as injection (e.g., intravenous, intraarterial, subcutaneous, intraperitoneal, or intramuscular injection, including instillation) and transdermal delivery, and can also be administered by oral, buccal, transnasal, transmucosal, or topical routes, or in the form of ophthalmic preparation, or by inhalation. Regarding these routes of administration, the pharmaceutical compositions of the present invention can be administered in suitable dosage forms. Said dosage forms include but are not limited to, tablets, capsules, lozenges, hard sugar agents, powders, sprays, creams, ointments, suppositories, gels, pastes, lotions, ointments, aqueous suspensions, injectable solutions, elixirs, and syrups. The term "vaccine" refers to preventive or therapeutic materials that provide at least one antigen or antigenic function. Antigen or antigenic function can stimulate the body's adaptive immune system to provide an adaptive immune response.

The term "excipient" as used herein refers to any substance, not itself a therapeutic agent, added to a formulation to improve its handling or storage properties or to permit or facilitate formation of a dose unit of the composition into a discrete article such as a capsule or tablet suitable for oral administration. Suitable excipients are well known to persons of ordinary skill in the art of manufacturing pharmaceutical formulations or food products. Excipients can include, by way of illustration and not limitation, buffers, diluents, disintegrants, binding agents, adhesives, wetting agents, polymers, lubricants, glidants, and substances added to mask or counteract a disagreeable taste or odor, flavors, dyes, fragrances, and substances added to improve appearance of the composition. Acceptable excipients include citrate buffer, phosphate buffer, acetate buffer, bicarbonate buffer, stearic acid, magnesium stearate, magnesium oxide, sodium and calcium salts of phosphoric and sulfuric acids, magnesium carbonate, talc, gelatin, acacia gum, sodium alginate, pectin, dextrin, mannitol, sorbitol, lactose, sucrose, starches, gelatin, cellulosic materials (such as cellulose esters of alkanoic acids and cellulose alkyl esters), low melting wax cocoa butter, amino acids, urea, alcohols, ascorbic acid, phospholipids, proteins (for example, serum albumin), ethylenediamine tetraacetic acid (EDTA), dimethyl sulfoxide (DMSO), sodium chloride or other salts, liposomes, mannitol, sorbitol, glycerol or powder, polymers (such as polyvinyl-pyrrolidone, polyvinyl alcohol, and polyethylene glycols), and other pharmaceutically acceptable materials.

The term "adjuvant" or "adjuvant component" is typically a (e.g., pharmacological or immunological) agent or composition that may modify (e.g., enhance) the efficacy of other agents (e.g., drugs or vaccines). Conventionally the term refers in the context of the invention to a compound or composition that serves as an auxiliary substance for immunogens and/or other pharmaceutically active compounds. It is to be interpreted in a broad sense and refers to a broad spectrum of substances that are able to increase the immunogenicity of antigens incorporated into or co-administered with an adjuvant in question. In the context of the present invention an adjuvant will preferably enhance the specific immunogenic effect of the active agents of the present invention. Typically, "adjuvant" or "adjuvant component" has the same meaning and can be used mutually. Adjuvants may be divided, e.g., into immune potentiators, antigenic delivery systems or even combinations thereof.

The term "delivery" refers to delivering an entity to the target, for example, delivering active agents, drugs, therapeutic agents and/or prophylactic agents to subjects, said subjects being tissues and/or cells of human and/or other animals.

The term "delivery vehicle" refers to a substance that can be used to administer or deliver one or more agents to a cell, a tissue, or a subject, particularly a human subject, with or without the agent(s) to be delivered. A delivery vehicle may preferentially deliver agent(s) to a particular subset or a particular type of cell. The selective or preferential delivery achieved by the delivery vehicle can be achieved by the properties of the vehicle or by a moiety conjugated to, associated with, or contained in the delivery vehicle, said moiety specifically or preferentially binding to a particular subset of cells. A delivery vehicle can also increase the in vivo half-life of the agent to be delivered, the efficiency of delivering the agent compared to delivery without using the delivery vehicle, and/or the bioavailability of the agent to be delivered.

As used herein, the term "nucleic acid" is meant to include any oligonucleotide or polynucleotide. Fragments containing up to 20 nucleotides are generally termed oligonucleotides, and longer fragments are called polynucleotides. In particular embodiments, oligonucletoides of the invention are 20-50 nucleotides in length.

In the context of this disclosure, the terms "polynucleotide" and "oligonucleotide" refer to a polymer or oligomer of nucleotide or nucleoside monomers consisting of naturally occurring bases, sugars and intersugar (backbone) linkages. The terms "polynucleotide" and "oligonucleotide" also includes polymers or oligomers comprising non-naturally occurring monomers, or portions thereof, which function similarly. Such modified or substituted oligonucleotides are often preferred over native forms because of properties such as, for example, enhanced cellular uptake and increased stability in the presence of nucleases.

Oligonucleotides are classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose.

The nucleic acid that is present in a lipid-nucleic acid particle according to this disclosure includes any form of nucleic acid that is known. The nucleic acids used herein can be single-stranded DNA or RNA, or double-stranded DNA or RNA, or DNA-RNA hybrids. Examples of double-stranded DNA include structural genes, genes including control and termination regions, and self-replicating systems such as viral or plasmid DNA. Examples of double-stranded RNA include siRNA and other RNA interference reagents. Single-stranded nucleic acids include, e.g., antisense oligonucleotides, ribozymes, microRNA, and triplex-forming oligonucleotides.

The term "RNA" refers to ribonucleic acid that may be naturally or non-naturally occurring. For example, an RNA may include modified and/or non-naturally occurring components such as one or more nucleobases, nucleosides, nucleotides, and linkers. An RNA may include a cap structure, a chain terminating nucleoside, a stem loop, a polyA sequence, and/or a polyadenylation signal. An RNA may have a nucleotide sequence encoding a polypeptide of interest. For example, an RNA may be a messenger RNA (mRNA). Translation of an mRNA encoding a particular polypeptide, for example, *in vivo* translation of an mRNA inside a mammalian cell, may produce the encoded polypeptide. RNAs may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), messenger RNA (mRNA, including cas9 mRNA), guide RNA (gRNA, also called single-guide RNA (sgRNA)), miRNA inhibitor (antagomir), ribozyme, and mixtures thereof. The antisense nucleic acid and siRNA can inhibit the expression of target gene and target protein in vitro or in vivo.

The term "modulating the expression of a target gene" refers to the ability of nucleic acids to silence, reduce or inhibit the expression of a target gene. To examine the extent of gene silencing, a test sample (e.g., a sample of cells in culture expressing the target gene) is contacted with nucleic acids that inhibit the expression of the target gene. The expression of the target gene in the test sample or test animal is compared to expression of the target gene in a control sample (e.g., a sample of cells in culture expressing the target gene) which is not contacted with or administered the nucleic acids. The expression of the target gene in the control sample may be assigned a value of 100%. In particular embodiments, modulating the expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%.

The term "transfecting" or "transfection" refers to the introduction of a species (e.g., an RNA) into a cell. Transfection may occur, for example, *in vitro, ex vivo,* or *in vivo.*

The term "PEGylation" is the process of both covalent and non-covalent attachment or amalgamation of polyethylene glycol (PEG, in pharmacy called macrogol) polymer chains to molecules and macrostructures, such as a drug, therapeutic protein or vesicle, which is then described as "PEGylated."

The present disclosure presents amine-containing lipids that are highly valuable for formulating lipid nanoparticle (LNP) compositions. These LNP compositions offer advantageous characteristics for efficiently delivering nucleic acid cargo, such as CRISPR/Cas gene editing components, into cells.

The present disclosure provides a lipid of Formula (I), 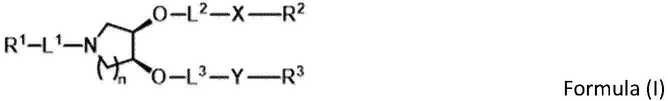 or pharmaceutically acceptable salts, solvates, hydrates, polymorphs, tautomers, stereoisomers, or isotopically enriched derivatives thereof,
wherein:
n is 1 or 2;
R¹ is amino, monoalkylamino, dialkylamino, hydroxy, alkoxy, carbamoyl, monoalkylcarbamoyl, dialkylcarbamoyl, pyrrolidinyl, piperidyl or morpholinyl;
R² and R³ are each independently an alkyl, alkenyl or alkynyl having 5 to 30 carbon atoms; L¹ is an alkyl having 2 to 6 carbon atoms;
L² and L³ are each independently an alkyl having 2 to 9 carbon atoms; and
X and Y are each independently -C(O)-O- or -O-C(O)-.

In some embodiments, R² and R³ may be the same as or different from each other. All the alkyl, alkenyl and alkynyl may be linear or branched, and may have 5, 6, 7, 8, 9, 10, 11, 12, 13, 14, 15, 16, 17, 18, 19, 20, 21, 22, 23, 24, 25, 26, 27, 28, 29 or 30 carbon atoms.

In some embodiments, L¹ may be an alkyl having 2, 3, 4, 5, or 6 carbon atoms.

In some embodiments, L² and L³ may be the same as or different from each other. The alkyl may be linear or branched, and may have 2, 3, 4, 5, 6, 7, 8, or 9 carbon atoms.

In some embodiments, X and Y may be the same as or different from each other.

In some embodiments, n is 1; R¹ is hydroxy; R² and R³ are each independently - CH((CH₂)ₚCH₃)((CH₂)_{q}CH₃) or -(CH₂)ₘCH((CH₂)ₚCH₃)((CH₂)_{q}CH₃), wherein m is 1, 2 or 3, p and q are each independently 1, 2, 3, 4, 5, 6, 7, 8, 9, 10, 11, or 12; L¹ is an alkyl having 2 to 4 carbon atoms; L² and L³ are each independently an alkyl with 3 to 6 carbons atoms; and X and Y are each independently -C(O)-O- or -O-C(O)-.

In some embodiments, n is 1; R¹ is hydroxy; R² and R³ are each independently - CH((CH₂)ₚCH₃)((CH₂)_{q}CH₃) or -CH₂CH((CH₂)ₚCH₃)((CH₂)_{q}CH₃), wherein p and q are each independently 3, 4, 5, 6, 7, 8, or 9; L¹ is an alkyl having 2 to 6 carbon atoms; L² and L³ are each independently an alkyl with 2 to 6 carbons atoms; and X and Y are each independently -C(O)-O- or -O-C(O)-.

In some embodiments, the lipid or pharmaceutically acceptable salts, solvates, hydrates, polymorphs, tautomers, stereoisomers, or isotopically enriched derivatives thereof of the present disclosure may be used as a constituent lipid of a lipid membrane structure for encapsulating an active agent. For example, the lipid of the present disclosure may be used in liposomes or lipids nanoparticles (LNPs) for nucleic acid encapsulation and delivery.

In some embodiments, the lipid of the present disclosure may be ionizable. In some embodiments, the lipid of the present disclosure is an ionizable cationic lipid, which is positively charged at low pH (thus binds nucleic acid, such as RNA) and becomes neutral at physiological pH which helps reduce the toxicity of LNP-RNA complexes *in vivo.* The lipid may serve two key functions including facilitating nucleic acid encapsulation in LNPs and mediating endosomal membrane disruption to enable nucleic acid release to the cytosol. In some embodiments, the lipid may enable endosomal uptake directly through interaction between its positive charge with the negatively charged cell membranes, or through binding to plasma proteins that support cellular uptake.

In some embodiments, the lipid may be deprotonated under neutral conditions and may be positively charged in pH conditions below its acid-dissociation constant (pKₐ). The pKₐ value is supposed to be sufficiently high so that at low pH the lipid is positively charged which enable binding with negatively charged RNA molecules and the formation of LNPs. Thus, at low endosomal pH, positive charge of ionizable amine-containing lipids allows interactions with endogenous anionic lipids, thereby leading to disruption of endosomal structure and release of LNPs cargo into cytoplasm. Simultaneously, the pKₐ value of the ionizable amine-containing lipids should be sufficiently low so that at physiological pH, the surface charge of LNPs will remain relatively neutral. This allows modulation of toxicity and immunogenicity of resulting LNPs and increases their circulation time. In some embodiments, the pKₐ of the protonated form of the lipid is about 5.0 to about 9.0, such as about 5.5, about 6.0, about 6.5, about 7.0 or greater, or about 8.5, about 8.0, about 7.5, about 7.0 or less. The pKa value of the lipid may be critical for efficient LNP delivery and transfection potency.

The present disclosure further provides an active agent delivery vehicle including the lipid or pharmaceutically acceptable salts, solvates, hydrates, polymorphs, tautomers, stereoisomers, or isotopically enriched derivatives thereof.

In some embodiments, the active agent delivery vehicle may be a drug delivery system, such as liposomes, single emulsions, micelles, nanoemulsions, lipids nanoparticles (LNPs), nanostructured lipid carriers (NLC), and a self-emulsifying drug delivery system (SEDDS). In some embodiments, the active agent delivery vehicle may be prepared at an acidic pH (about 4 or 5) lower than the pKa value of the lipid, followed by a buffer/pH exchange step.

In some embodiments, the active agent delivery vehicle further includes one or more neutral lipids, steroid lipids, and/or PEGylated lipids. In some embodiments, the active agent delivery vehicle includes at least one neutral lipid, at least one steroid lipid, and at least one PEGylated lipid. These constituents may facilitate monodisperse nanoparticle formation, improve nanoparticle stability, enable efficient nucleic acid encapsulation, aid cellular uptake, and promote endosomal escape of nucleic acid cargo.

In some embodiments, the neutral lipids are also called helper lipids or structural lipids. The neutral lipid may be any lipid substance which is uncharged or exists in the form of neutral zwitterion at the chosen pH. In some embodiments, the neutral lipids may be phospholipids, such as 1,2-dilinoleoyl-sn-glycero-3-phosphocholines (DLPC), 1,2-dimyristoleoyl-sn-glycero-3-phosphocholines (DMPC), 1,2-dioleoyl-sn-glycero-3-phosphocholines (DOPC), 1,2-dipalmitoyl-sn-glycero-3-phosphocholines (DPPC), 1,2-distearoyl-sn-glycero-3-phosphatidylcholines (DSPC), 1,2-diundecanoyl-sn-glycero-3-phosphatidylcholines (DUPC), 1-plamitoyl-2-oleoyl-sn-glycero-3-phosphocholines (POPC), 1,2-di-O-octadecenyl-sn-glycero-3-phosphatidylcholines (18:0 Diether PC), 1-oleoyl-2-cholesterylhemisuccinyl-sn-glycero-3-phosphocholines (OChemsPC), 1-O-hexadecyl-sn-glycero-3-phosphatidylcholines (C16 Lyso PC), 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines (DOPE), 1,2-diphytanyl-sn-glycero-3-phosphoethanolamines (ME 16.0 PE), 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3-phosphoethanolamines, 1,2-dioleoyl-sn-glycero-3-phospho-rac-(1-glycerol) sodium salts (DOPG), dioleoyl phosphatidylserines (DOPS), dipalmitoylphosphatidylglycerols (DPPG), palmitoyloleoyl phosphatidylethanolamines (POPE), distearoyl phosphatidylethanolamines (DSPE), dipalmitoyl phosphatidylethanolamines (DPPE), dimyristoleoyl phosphoethanolamines (DMPE), 1-stearoyl-2-oleoyl-stearoylethanolamines (SOPE), 1-stearoyl-2-oleoyl-phosphatidylcholines (SOPC), sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, lysophosphatidylethanolamines (LPE), and combinations thereof. The neutral lipid may be synthetic or natural. In some embodiments, the preferred phospholipids for LNPs are DSPC and DOPE.

The steroid lipids may improve cell entry of an active agent encapsulated in the delivery vehicle. Examples of the steroid lipids include cholesterol, coprostanol, sitosterol, ergosterol, campesterol, stigmasterol, brassicasterol tomatidine, ursolic acid, α-tocopherol, and combinations thereof, but are not limited thereto.

In some embodiments, the PEGylated lipids are molecules containing both lipid and PEG moieties, which are also called polyethylene glycol (PEG)-lipid conjugates. The PEGylated lipids may improve stability and circulation time of the delivery vehicle by preventing serum protein binding. In some embodiments, the PEGylated lipids may be 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), PEG-dialkyloxypropyl (PEG-DAA), and combinations thereof. Specifically, the PEGylated lipids may be one or more of PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-dioleoylphosphatidylethanolamine, PEG2000-1,2-dioleoylphosphatidylethanolamine, PEG2000-2,3-distearoylglycerol (PEG-DMG), and 1,2-dimyristoyl-rac-glycero-3-methoxypolyethylene glycol-2000 (DMG-PEG2000).

The present disclosure also provides a composition comprising the active agent delivery vehicle, and an active agent encapsulated by the active agent delivery vehicle.

In some embodiments, the composition is a pharmaceutical composition which may be used as a drug for treating any disease (such as cancer and malignant tumors), anti-infectious agents, antiviral agents, antifungal agents, and vaccines.

In some embodiments, the active agent is encapsulated in the active agent delivery vehicle. In some embodiments, the active agent includes one or more nucleic acids. Examples of the nucleic acids include DNA, RNA, antisense nucleic acid, plasmid, interfering nucleic acid, aptamer, small interfering RNA (siRNA), asymmetrical interfering RNA (aiRNA), microRNA (miRNA), Dicer-substrate RNA (dsRNA), small hairpin RNA (shRNA), mRNA (messenger RNA), guide RNA (gRNA), miRNA inhibitor (antagomir), ribozyme, and combinations thereof. In some embodiments, the mRNA encodes an enzyme, an antigen, an antibody or an antigen binding fragment.

In some embodiments, the pharmaceutical composition further includes one or more pharmaceutically acceptable excipients or adjuvants. In some embodiments, the pharmaceutically acceptable excipients may include deionized water, ultrapure water, phosphate buffer, or physiological saline, more preferably phosphate buffer or physiological saline.

The present disclosure further provides a method for treating or preventing a disease or disorder, comprising administering the pharmaceutical composition to a subject in need thereof.

In some embodiments, the pharmaceutical composition according to the disclosure is administered topically or systemically by any method known in the art, including, but not limited to, intramuscular, intradermal, intravenous, subcutaneous, intraperitoneal, intranasal, oral, mucosal or external routes. The appropriate route, formulation and administration schedule can be determined by those skilled in the art. In the present disclosure, the pharmaceutical composition can be formulated in various ways, according to the corresponding route of administration, such as a liquid solution, a suspension, an emulsion, a syrup, a tablet, a pill, a capsule, a sustained release formulation, a powder, a granule, an ampoule, an injection, an infusion, a kit, an ointment, a lotion, a liniment, a cream or a combination thereof. If necessary, it may be sterilized or mixed with any pharmaceutically acceptable excipient, many of which are known to one of ordinary skill in the art.

The external route as used herein is also known as local administration, which includes but is not limited to administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulization, suppositories, pessaries, retention enemas and chewable or suckable tablets or pellets or liposome or microencapsulation preparations.

The present disclosure further provides a method for introducing a nucleic acid into a target cell or transfecting the target cell with the nucleic acid, comprising bringing the composition into contact with the target cell. In some embodiments, the active agent delivery vehicle encapsulating the nucleic acid may be administered to a subject, such that the nucleic acid-introducing vehicle can contact with the target cell of the subject.

In another aspect, the present disclosure provides a method of modulating the expression of a target gene in a cell, comprising providing to the cell the composition as disclosed herein.

The following Examples are given for the purpose of illustration only and are not intended to limit the scope of the present disclosure.

### SYNTHESIS OF EXAMPLES

General procedure of synthesis ionizable amine-containing lipids

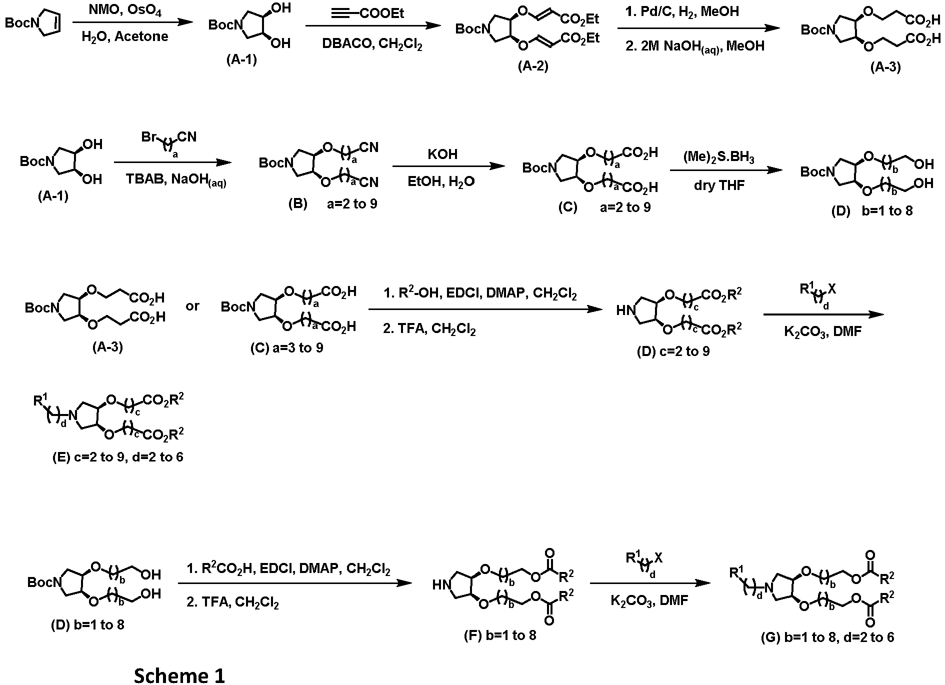

### Synthesis of tert-butyl (3S,4R)-3,4-dihydroxypyrrolidine-1-carboxylate (A-1)

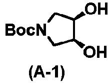

A solution of the tert-butyl 2,5-dihydro-1H-pyrrole-1-carboxylate (2875 mg; 16.98 mmol) in acetone (30 mL) was added dropwise to a mixture of NMO (2188 mg; 18.68 mmol, 1.1 eq), osmium tetroxide (4% in water) (25 mg, 0.004 mmol) and water (30 mL) while keeping the temperature below 25°C. The reaction mixture was then stirred for 16 hours at ambient temperature. Acetone was evaporated off and the organic phase was extracted with ethyl acetate. The combined organic phases were washed in turn with water and brine, and dried over magnesium sulphate. The solvent was evaporated under vacuum, and the residue was a crude product (A-1) (2865 mg, yield: 83%), appearing as a brown oil, which was utilized in the next reaction without further purification. ¹H NMR (600 MHz, CDCl₃) δ 4.30 - 4.21 (m, 2H), 3.60 (ddt, J = 15.8, 11.4, 5.3 Hz, 2H), 3.36 (ddd, J = 27.5, 11.6, 4.0 Hz, 2H), 2.77 (b, 1H), 1.67 (b, 1H),1.47 (d, J = 2.1 Hz, 9H).

### Synthesis of diethyl 3,3'-(((3S,4R)-1-(tert-butoxycarbonyl)pyrrolidine-3,4-diyl) bis(oxy))(2E,2'E)-diacrylate (A-2)

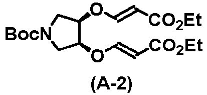

The crude product (A-1) (2070 mg, 10.08 mmol) and DABCO (340 mg, 3.02 mmol, 0.3 eq) were dissolved in 20 mL of dry DCM. This solution was then slowly added drop by drop to a mixture of ethyl propiolate (5936 mg, 60.51 mmol, 6.0 eq) in 25 mL of dry DCM, under nitrogen atmosphere in an ice/water bath. After the reaction was completed, NH₄Cl_{(aq)}/EA was added for extraction, and the organic layer was taken, dried using MgSO₄ dried, and concentrated and pumped to dryness. The dried product was purified by chromatography on a column of silica gel using a gradient of hexanes/EtOAc (3/2 to 2/3), and then concentrated in vacuo to obtain a light yellow oily product (A-2) (1757 mg, yield: 43%).¹H NMR (600 MHz, CDCl₃) δ 7.51 (d, J = 12.5 Hz, 2H), 5.33 (d, J = 12.6 Hz, 2H), 4.61 (h, J = 3.3, 2.8 Hz, 2H), 4.22 - 4.17 (m, 4H), 3.76 (dq, J = 11.6, 6.8, 5.9 Hz, 2H), 3.58 (dd, J = 11.8, 4.3 Hz, 1H), 3.53 - 3.48 (m, 1H), 1.48 (dd, J = 3.3, 1.4 Hz, 9H), 1.31 - 1.28 (m, 6H); LCMS(ESI) :m/z Calcd for C₁₉H₂₉NO₈ + H]⁺ 399.44, found 399.9.

### Synthesis of 3,3'-(((3S,4R)-1-(tert-butoxycarbonyl)pyrrolidine-3,4-diyl)bis(oxy)) dipropionic acid (A-3)

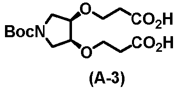

A solution of (A-2) (1700 mg, 4.25 mmol) and 10% Pd-C (170 mg) in a solvent mixture of ethyl acetate and methanol at a volume ratio of 5:1 were added into a round bottom bottle under hydrogen condition and reacted at r.t for 4 Hr. The product was filtered with celite, and the organic layer was taken and concentrated to dryness to obtain a colorless oily product.

A solution of the above crude product in MeOH/H₂O (1/1, 40 mL) and 2M NaOH (6 mL) were added into a round bottom bottle and reacted at r.t for 16 Hr. After the reaction was completed, a part of the solvent was drained, and the residual solution was cooled in an ice bath then adjusted to pH 4 with 2N HCl. The resulting solution was extracted with EtOAc twice and the combined organic layers were dried (MgSO₄). The dried product was filtered and concentrated in vacuo to provide crude (A-3) as a viscous colorless oily product (1123 mg, yield: 76%).¹H NMR (600 MHz, CDCl₃) δ 4.20 - 4.11 (m, 1H), 3.99 (dd, J = 22.2, 4.3 Hz, 2H), 3.82 (ddd, J = 13.9, 7.7, 3.2 Hz, 4H), 3.57 - 3.46 (m, 2H), 3.38 - 3.30 (m, 1H), 2.75 - 2.55 (m, 4H), 1.49 - 1.46 (m, 9H). LCMS(ESI):m/z Calcd for C₁₉H₃₃NO₈ +H]⁺ 403.47; found 404.0 [M+H]⁺.

### Synthesis of tert-butyl (3S,4R)-3,4-bis(4-cyanobutoxy)pyrrolidine-1-carboxylate (A-4)

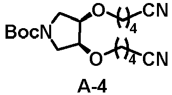

The crude product of (A-1) (1900mg, 9.35 mmol), 5-bromopentanenitrile (6057 mg, 37.39 mmol, 4.0eq), and TBAB (605mg, 1.87mmol, 0.2eq), were mixed in 40W% NaOH(6 mL) in a round bottom bottle. The mixture was warmed to 65 °C and stirred for reaction for 24 h. The mixture was cooled in an ice-water bath and diluted with water (20 mL), then adjusted to pH=5~6 with 12N HCl. The resulting solution was extracted with EtOAc twice, and the combined organic layers were dried (MgSO₄). The dried product was filtered and concentrated in vacuo to provide a crude product. The crude product was purified by CombiFlash MPLC using a gradient of Hexanes/EtOAC (EtOAC= 28~31%), and then concentrated in vacuo to obtain a colorless oily product (A-4) (1776 mg, yield: 52%).¹H NMR (600 MHz, CDCl₃) δ 3.92 (q, J = 4.8, 4.2 Hz, 2H), 3.63 - 3.46 (m, 6H), 3.44 (dd, J = 11.5, 3.8 Hz, 1H), 3.32 (dd, J = 10.8, 5.2 Hz, 1H), 2.51 - 2.35 (m, 4H), 1.78 (tq, J = 10.3, 6.0 Hz, 8H), 1.48 (d, J = 0.7 Hz, 9H); LCMS(ESI):m/z Calcd for:C₁₉H₃₁N₃O+H]⁺ 365.47, found 366.3 [M+H]⁺.

### Synthesis of 5,5'-(((3S,4R)-1-(tert-butoxycarbonyl)pyrrolidine-3,4-diyl)bis(oxy)) dipentanoic acid (A-5)

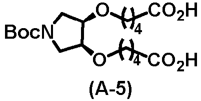

The product (A-4) (1300 mg, 3.55 mmol) and KOH (1944 mg, 35.54 mmol, 10 eq) were mixed in EtOH/H₂O (1/1, 20mL) in a round bottom bottle. The mixture was warmed to 110 °C and stirred for reaction for 32 h. The mixture was then cooled down to room temperature, and EtOH was evaporated off. The resulting mixture was cooled in an ice-water bath and diluted with water (10 mL), then adjusted to pH 4~5 with 36N HCl. The resulting solution was extracted with EtOAc twice and the combined organic layers were dried (MgSO₄). The dried product was filtered and concentrated in vacuo to obtain as a viscous colorless product (A-5) (1219 mg, yield: 85%). ¹H NMR (600 MHz, CDCl₃) δ 3.96 - 3.86 (m, 2H), 3.64 - 3.43 (m, 7H), 3.34 (dd, J = 11.0, 5.5 Hz, 1H), 2.41 (dq, J = 7.4, 4.1, 3.0 Hz, 4H), 1.76 (d, J = 15.0 Hz, 4H), 1.68 (dq, J = 12.5, 6.3 Hz, 4H), 1.47 (s, 9H). LCMS(ESI):m/z Calcd for C₁₉H₃₃NO₈ +H]⁺ 403.47, found 404.1 [M+H]⁺.

### Synthesis of tert-butyl (3S,4S)-3,4-bis((5-hydroxypentyl)oxy) pyrrolidine-1-carboxylate (A-6)

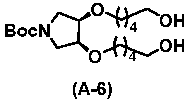

To a solution of (A-5) (600 mg, 1.49 mmol) in dry THF (10 mL), cooled in an ice water bath under nitrogen, Borane dimethylsulfide (2M in THF) (2.9 mL, 4.0 eq) was added over a period of 5 min. The mixture was warmed to 65 °C and then stirred for 3h. The mixture was then cooled in an ice-water bath under nitrogen, and MeOH (10 mL) was added into the mixture carefully and slowly over a period of 5 min. The mixture was again warmed to 65 °C and then stirred for 15min. The mixture was then cooled down to room temperature, and the solvent was evaporated off. The residue was extracted with EtOAc twice and the combined organic layers were dried (MgSO₄). The dried product was filtered and concentrated in vacuo to obtain as a viscous colorless product (A-6) (497 mg, yield: 89%). ¹H NMR (600 MHz, CDCl₃) δ 3.85 (ddt, J = 4.8, 3.1, 1.8 Hz, 2H), 3.67 (tt, J = 6.7, 2.6 Hz, 4H), 3.56 - 3.42 (m, 7H), 3.38 - 3.31 (m, 1H), 1.60 (td, J = 7.1, 6.7, 2.9 Hz, 8H), 1.48 (s, 9H), 1.46 - 1.40 (m, 4H); LCMS(ESI):m/z Calcd for C₁₉H₃₇NO₆ +H]⁺ 375.51, found 376.0 [M+H]⁺.

### Synthesis of di(heptadecan-9-yl) 5,5'-(((3S,4R)-pyrrolidine-3,4-diyl)bis(oxy)) dipentanoate (A-7)

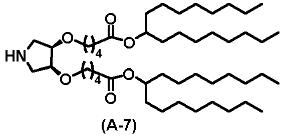

To a suspension of (A-5) (730 mg, 1.81 mmol) in dry DCM (10 mL), EDC-HCl (1038 mg, 5.43 mmol, 3.0eq), 9-Heptadecanol (925 mg, 3.62mmol, 2.0eq), DIPEA (1403 mg, 10.86 mmol, 6.0eq), and DMAP (88mg, 0.72 mmol, 0.4 eq) were sequentially added. The mixture was stirred at room temperature under nitrogen overnight, and then was cast into water and extracted with DCM twice. The organic layer was separated, washed with brine, and dried using MgSO₄. The dried product was filtered and concentrated in vacuo to obtain a crude product. The crude product was purified by CombiFlash MPLC using a gradient of Hexane/EtOAc (EtOAc= 8~13%), and then concentrated in vacuo to obtain a colorless oily product. To a solution of the crude product (1140 mg, 1.29 mmol) in dry dichloromethane (15 mL), TFA (1.8 mL) was added. The mixture was stirred at room temperature under nitrogen for 1.5 h. The mixture was cooled in an ice-water bath and diluted with DCM (10 mL), and then adjusted to pH 8 with NaHCO_{3(aq)}. The resulting solution was extracted with DCM and the combined organic layers were dried (MgSO₄). The dried product was filtered and concentrated in vacuo to obtain as a colorless oily product (A-7) (776 mg, yield: 55%). ¹H NMR (600 MHz, CDCl₃) δ 4.88 (p, J = 6.3 Hz, 2H), 4.07 (q, J = 3.9, 3.3 Hz, 2H), 3.61 (dt, J = 9.0, 6.1 Hz, 2H), 3.56 - 3.51 (m, 2H), 3.44 (dd, J = 11.6, 5.0 Hz, 2H), 3.31 (dd, J = 11.8, 4.7 Hz, 2H), 2.37 - 2.30 (m, 4H), 1.75 - 1.69 (m, 4H), 1.67 - 1.61 (m, 4H), 1.52 (d, J = 6.3 Hz, 8H), 1.33 - 1.24 (m, 48H), 0.90 (t, J = 7.0 Hz, 12H); LCMS(ESI):m/z Calcd for C₄₈H₉₃NO₆ +H]⁺ 780.27, found 781.0 [M+H]⁺.

### Synthesis of (((3S,4R)-pyrrolidine-3,4-diyl)bis(oxy))bis(pentane-5,1-diyl) bis(2-hexyldecanoate) (A-8)

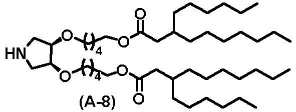

The synthetic steps are the same as described in (A-7). ¹H NMR (600 MHz, CDCl₃) δ 4.09 (t, J = 6.8 Hz, 4H), 4.01 (q, J = 4.0 Hz, 2H), 3.58 (dt, J = 9.1, 6.5 Hz, 2H), 3.51 (dt, J = 9.0, 6.5 Hz, 2H), 3.33 (dd, J = 11.7, 5.0 Hz, 2H), 3.23 (dd, J = 11.8, 4.5 Hz, 2H), 2.33 (tt, J = 8.9, 5.3 Hz, 2H), 1.63 (ddd, J = 15.9, 10.6, 7.2 Hz, 12H), 1.49 - 1.41 (m, 8H), 1.33 - 1.23 (m, 40H), 0.90 (td, J = 7.0, 1.6 Hz, 12H); LCMS(ESI):m/z Calcd for C₄₆H₈₉NO₆ +H]⁺ 752.22, found 753.0 [M+H]⁺.

### Example 1

### di(heptadecan-9-yl)3,3'-(((3R,4S)-1-(2-hydroxyethyl)pyrrolidine-3,4-diyl)bis(oxy))dipropionate

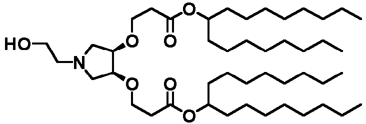

¹H NMR (600 MHz, CDCl₃) δ 4.94 - 4.85 (m, 2H), 3.97 (ddt, J = 7.8, 5.5, 2.8 Hz, 2H), 3.87 - 3.74 (m, 4H), 3.61 (dd, J = 6.2, 4.4 Hz, 2H), 3.07 - 3.00 (m, 2H), 2.72 - 2.67 (m, 2H), 2.66 - 2.56 (m, 6H), 1.57 - 1.48 (m, 8H), 1.36 - 1.22 (m, 48H), 0.90 (t, J = 7.0 Hz, 12H); LCMS (ESI): 768.6[M+H]⁺.

### Example 2

### di(heptadecan-9-yl)3,3'-((3R,4S)-1-(3-hydroxypropyl)pyrrolidine-3,4-diyl)bis(oxy))propionate

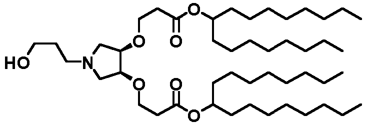

¹H NMR (600 MHz, CDCl₃) δ 4.96 - 4.83 (m, 2H), 3.93 (ddt, J = 8.0, 5.5, 2.8 Hz, 2H), 3.86 - 3.70 (m, 6H), 3.18 (dd, J = 10.0, 5.4 Hz, 2H), 2.77 (t, J = 5.8 Hz, 2H), 2.65 - 2.50 (m, 6H), 1.75 - 1.62 (m, 2H), 1.53 (dd, J = 10.4, 5.1 Hz, 8H), 1.36 - 1.21 (m, 48H), 0.90 (t, J = 7.0 Hz, 12H). LCMS (ESI): 783.4 [M+H]⁺.

### Example 3

### di(heptadecan-9-yl)5,5'-(((3S,4R)-1-(3-hydroxypropyl)pyrrolidine-3,4-diyl)bis(oxy))dipentanoate

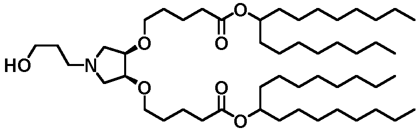

¹H NMR (600 MHz, CDCl₃) δ 4.88 (p, J = 6.2 Hz, 2H), 3.88 (t, J = 4.3 Hz, 2H), 3.83 - 3.78 (m, 2H), 3.56 (dt, J = 9.2, 6.3 Hz, 2H), 3.47 (dt, J = 9.2, 6.3 Hz, 2H), 3.20 (s, 2H), 2.81 (s, 2H), 2.61 - 2.51 (m, 2H), 2.33 (t, J = 7.4 Hz, 4H), 1.72 (ddd, J = 9.0, 6.7, 4.5 Hz, 6H), 1.64 (dd, J = 8.5, 5.7 Hz, 4H), 1.52 (d, J = 6.0 Hz, 8H), 1.35 - 1.22 (m, 48H), 0.90 (t, J = 7.0 Hz, 12H); LCMS(ESI): 838.9 [M+H]⁺.

### Example 4

### di(heptadecan-9-yl)5,5'-(((3S,4R)-1-(2-hydroxyethyl)pyrrolidine-3,4-diyl)bis(oxy))dipentanoate

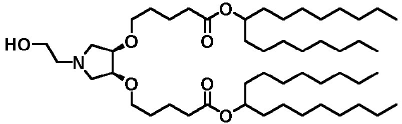

¹H NMR (600 MHz, CDCl₃) δ 4.88 (p, J = 6.3 Hz, 2H), 3.87 - 3.79 (m, 2H), 3.63 (t, J = 5.4 Hz, 2H), 3.47 (ddt, J = 26.6, 9.1, 6.3 Hz, 4H), 2.93 (dd, J = 9.9, 6.0 Hz, 2H), 2.68 (dt, J = 12.3, 5.5 Hz, 1H), 2.60 (ddd, J = 10.0, 8.9, 4.6 Hz, 3H), 2.33 (t, J = 7.4 Hz, 4H), 1.71 (dtd, J = 9.1, 7.9, 7.2, 5.9 Hz, 4H), 1.66 - 1.58 (m, 4H), 1.52 (d, J = 5.9 Hz, 8H), 1.36 - 1.22 (m, 48H), 0.90 (t, J = 7.0 Hz, 12H); LCMS (ESI): 825.1 [M+H]⁺.

### Example 5

### (((3S,4R)-1-(3-hydroxypropyl)pyrrolidine-3,4-diyl)bis(oxy))bis(pentane-5,1-diyl)bis(2 - hexyldecanoate)

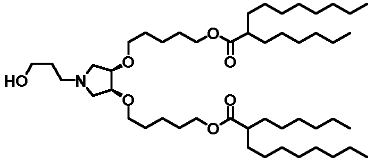

¹H NMR (600 MHz, CDCl₃) δ 4.08 (t, J = 6.7 Hz, 4H), 3.87 (d, J = 5.0 Hz, 2H), 3.82 - 3.77 (m, 2H), 3.54 (q, J = 7.6 Hz, 2H), 3.46 (q, J = 7.5 Hz, 2H), 3.26 - 3.13 (m, 2H), 2.79 (t, J = 5.7 Hz, 2H), 2.54 (dd, J = 10.4, 4.7 Hz, 2H), 2.32 (ddd, J = 8.9, 6.0, 2.8 Hz, 2H), 1.72 (q, J = 5.6 Hz, 2H), 1.64 (ddq, J = 26.2, 14.4, 7.6 Hz, 12H), 1.44 (d, J = 8.8 Hz, 8H), 1.27 (s, 40H), 0.90 (t, J = 7.0 Hz, 12H); LCMS (ESI): 810.9 [M+H]⁺.

### Example 6

### (((3S,4R)-1-(2-hydroxyethyl)pyrrolidine-3,4-diyl)bis(oxy))bis(pentane-5,1-diyl)bis(2-hexyldecanoate)

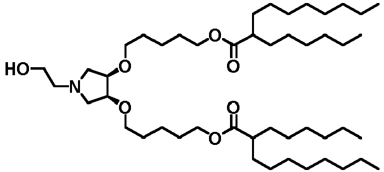

¹H NMR (600 MHz, CDCl₃) δ 4.09 (t, J = 6.7 Hz, 4H), 3.91 (t, J = 4.3 Hz, 2H), 3.62 (t, J = 5.3 Hz, 2H), 3.55 (dt, J = 9.3, 6.6 Hz, 2H), 3.47 (dt, J = 9.2, 6.7 Hz, 2H), 3.08 (dd, J = 9.7, 5.3 Hz, 2H), 2.71 (t, J = 5.3 Hz, 2H), 2.64 (dd, J = 9.7, 4.6 Hz, 2H), 2.32 (td, J = 9.1, 4.6 Hz, 2H), 1.68 - 1.63 (m, 12H), 1.49 - 1.39 (m, 8H), 1.34 - 1.22 (m, 40H), 0.90 (t, J = 6.9 Hz, 12H); LCMS (ESI): 796.4 [M+H]⁺.

### Example 7

### di(heptadecan-9-yl)3,3'-(((3R,4S)-1-(4-hydroxybutyl)pyrrolidine-3,4-diyl)bis(oxy))dipropionate

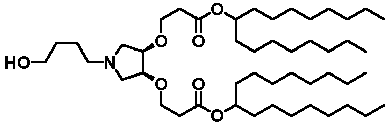

¹H NMR (600 MHz, CDCl₃) δ 4.90 (p, J = 6.3 Hz, 2H), 3.99 (s, 2H), 3.86 - 3.81 (m, 2H), 3.80 - 3.74 (m, 2H), 3.59 (s, 2H), 3.20 (s, 2H), 2.66 - 2.56 (m, 6H), 1.67 (s, 4H), 1.52 (t, J = 6.4 Hz, 8H), 1.35 - 1.23 (m, 50H), 0.90 (t, J = 6.9 Hz, 12H). LCMS (ESI): 796.9 [M+H]⁺.

### Example 8

### (((3R,4S)-1-(3-hydroxypropyl)pyrrolidine-3,4-diyl)bis(oxy))bis(butane-4,1-diyl)bis(2-hexyldecanoate)

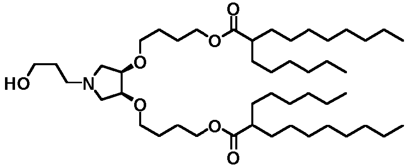

¹H NMR (600 MHz, CDCl₃) δ 4.11 (t, J = 6.5 Hz, 4H), 3.92 (t, J = 4.6 Hz, 2H), 3.83 - 3.78 (m, 2H), 3.59 (dt, J = 9.1, 6.2 Hz, 2H), 3.50 (dt, J = 9.1, 6.2 Hz, 2H), 3.29 (s, 2H), 2.87 (s, 2H), 2.63 (s, 2H), 2.33 (tt, J = 8.9, 5.4 Hz, 2H), 1.79 - 1.64 (m, 12H), 1.64 - 1.55 (m, 6H), 1.35 - 1.21 (m, 40H), 0.90 (td, J = 7.0, 1.8 Hz, 12H). LCMS (ESI): 782.8 [M+H]⁺.

### Example 9

### (((3S,4R)-1-(5-hydroxypentyl)pyrrolidine-3,4-diyl)bis(oxy))bis(pentane-5,1-diyl)bis(2-hexyldecanoate)

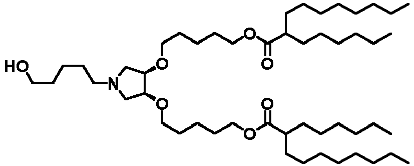

¹H NM(600 MHz, CDCl₃) δ 4.09 (t, J = 6.7 Hz, 4H), 3.67 (t, J = 6.3 Hz, 2H), 3.60 (s, 2H), 3.49 (q, J = 7.2 Hz, 2H), 2.33 (tt, J = 9.0, 5.3 Hz, 2H), 1.69 - 1.58 (m, 24H), 1.44 (dt, J = 17.7, 6.7 Hz, 10H), 1.35 - 1.20 (m, 40H), 0.90 (td, J = 7.0, 1.5 Hz, 12H). LCMS (ESI): 839.0 [M+H]⁺.

### Example 10

### (((3S,4R)-1-(6-hydroxyhexyl)pyrrolidine-3,4-diyl)bis(oxy))bis(pentane-5,1-diyl)bis(2-hexyldecanoate)

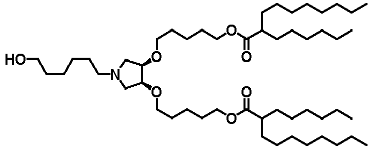

¹H NMR (600 MHz, CDCl₃) δ 4.08 (t, J = 6.7 Hz, 4H), 3.93 (s, 2H), 3.66 (t, J = 6.5 Hz, 2H), 3.61 - 3.52 (m, 2H), 3.47 (dt, J = 9.3, 6.7 Hz, 2H), 2.33 (tt, J = 8.9, 5.3 Hz, 2H), 1.71 - 1.55 (m, 20H), 1.48 - 1.36 (m, 12H), 1.34 - 1.22 (m, 40H), 0.90 (td, J = 7.1, 1.6 Hz, 12H). LCMS (ESI): 853.2 [M+H]⁺.

### Example 11

### di(heptadecan-9-yl)6,6'-(((3R,4S)-1-(3-hydroxypropyl)pyrrolidine-3,4-diyl)bis(oxy))dihexanoate

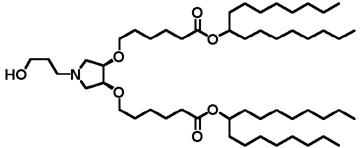

¹H NMR (600 MHz, CDCl₃) δ 4.88 (p, J = 6.2 Hz, 2H), 3.89 (q, J = 4.9, 4.4 Hz, 2H), 3.85 - 3.75 (m, 2H), 3.54 (qd, J = 6.8, 3.3 Hz, 2H), 3.45 (td, J = 8.5, 7.9, 6.0 Hz, 2H), 3.33 - 3.15 (m, 2H), 2.83 (s, 2H), 2.64 - 2.48 (m, 2H), 2.30 (t, J = 7.6 Hz, 4H), 1.73 (p, J = 5.5 Hz, 2H), 1.64 (ddd, J = 20.2, 14.7, 7.3 Hz, 8H), 1.52 (q, J = 6.6, 6.0 Hz, 8H), 1.40 (tt, J = 9.9, 6.3 Hz, 4H), 1.33 - 1.24 (m, 48H), 0.90 (t, J = 7.0 Hz, 12H). LCMS (ESI): 867.1 [M+H]⁺.

### Example 12

### di(heptadecan-9-yl)6,6'-(((3R,4S)-1-(5-hydroxypentyl)pyrrolidine-3,4-diyl)bis(oxy))dihexanoate

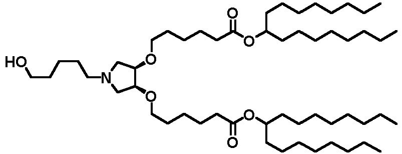

¹H NMR (600 MHz, CDCl₃) δ 4.88 (p, J = 6.3 Hz, 2H), 3.97 (s, 2H), 3.67 (t, J = 6.3 Hz, 2H), 3.57 (d, J = 8.1 Hz, 2H), 3.47 (dt, J = 9.0, 6.6 Hz, 2H), 2.31 (t, J = 7.6 Hz, 4H), 1.71 - 1.58 (m, 19H), 1.54-1.51(dd , 8H), 1.44 - 1.36 (m, 4H), 1.28 (d, J = 9.8 Hz, 48H), 0.90 (t, J = 7.0 Hz, 12H). LCMS (ESI): 895.1 [M+H]⁺.

### Example 13

### di(heptadecan-9-yl)6,6'-(((3R,4S)-1-(4-hydroxybutyl)pyrrolidine-3,4-diyl)bis(oxy))dihexanoate

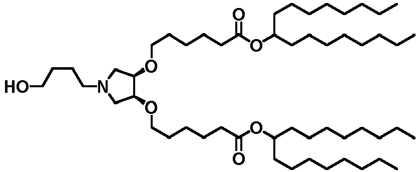

¹H NMR (600 MHz, CDCl₃) δ 4.93 - 4.82 (m, 2H), 3.96 - 3.85 (m, 2H), 3.58 (t, J = 4.7 Hz, 2H), 3.54 (dt, J = 9.4, 6.6 Hz, 2H), 3.45 (dt, J = 9.2, 6.7 Hz, 2H), 3.22 (d, J = 7.0 Hz, 2H), 2.66 - 2.58 (m, 2H), 2.53 (dd, J = 10.1, 4.9 Hz, 2H), 2.30 (t, J = 7.6 Hz, 4H), 1.72 - 1.59 (m, 12H), 1.52 (d, J = 6.3 Hz, 8H), 1.44-1.36 (m, 4H), 1.28 (d, J = 9.6 Hz, 48H), 0.90 (t, J = 7.0 Hz, 12H). LCMS (ESI): 881.2 [M+H]⁺.

### Example 14

### (((3S,4R)-1-(3-hydroxypropyl)pyrrolidine-3,4-diyl)bis(oxy))bis(hexane-6,1-diyl)bis(hexyldecanoate)

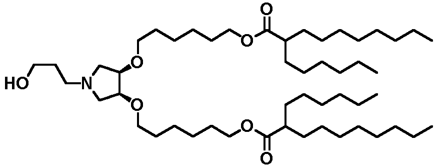

¹H NMR (600 MHz, CDCl₃) δ 4.08 (t, J = 6.7 Hz, 4H), 3.90 (q, J = 4.9, 4.4 Hz, 2H), 3.81 (t, J = 5.3 Hz, 2H), 3.54 (dt, J = 9.0, 6.6 Hz, 2H), 3.46 (dt, J = 9.1, 6.6 Hz, 2H), 3.28 (s, 2H), 2.86 (s, 2H), 2.65 - 2.55 (m, 2H), 2.33 (tt, J = 9.0, 5.3 Hz, 2H), 1.75 (p, J = 5.5 Hz, 2H), 1.61 (dddd, J = 16.6, 12.9, 10.0, 7.1 Hz, 12H), 1.48 - 1.41 (m, 4H), 1.39 (p, J = 3.7 Hz, 8H), 1.33 - 1.23 (m, 40H), 0.90 (td, J = 7.0, 1.8 Hz, 12H). LCMS (ESI): 839.0 [M+H]⁺.

### Example 15

### (((3S,4R)-1-(5-hydroxypentyl)pyrrolidine-3,4-diyl)bis(oxy))bis(hexane-6,1-diyl) bis(2-hexyldecanoate)

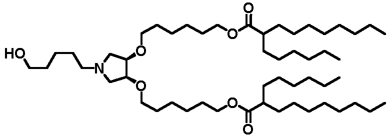

¹H NMR (600 MHz, CDCl₃) δ 4.08 (t, J = 6.7 Hz, 4H), 3.96 (s, 2H), 3.67 (t, J = 6.4 Hz, 2H), 3.57 (q, J = 7.2 Hz, 2H), 3.47 (dt, J = 9.1, 6.6 Hz, 2H), 2.33 (tt, J = 9.0, 5.3 Hz, 2H), 1.66 - 1.59 (m, 22H), 1.48 - 1.41 (m, 6H), 1.39 (p, J = 3.5 Hz, 8H), 1.33 - 1.22 (m, 40H), 0.90 (td, J = 7.0, 1.8 Hz, 12H). LCMS (ESI): 867.0 [M+H]⁺.

### Example 16

### (((3S,4R)-1-(4-hydroxybutyl)pyrrolidine-3,4-diyl)bis(oxy))bis(hexane-6,1-diyl)bis(2-hexyldecanoate)

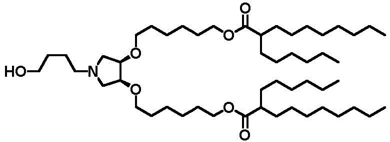

¹H NMR (600 MHz, CDCl₃) δ 4.08 (t, J = 6.7 Hz, 4H), 3.92 (t, J = 4.4 Hz, 2H), 3.59 (d, J = 5.1 Hz, 2H), 3.54 (dt, J = 9.2, 6.6 Hz, 2H), 3.46 (dt, J = 9.1, 6.6 Hz, 2H), 3.26 (s, 2H), 2.63 (s, 2H), 2.59 - 2.48 (m, 2H), 2.33 (tt, J = 9.0, 5.3 Hz, 2H), 1.72 - 1.55 (m, 16H), 1.45 (dddt, J = 11.5, 9.8, 6.0, 3.0 Hz, 4H), 1.39 (q, J = 3.7 Hz, 8H), 1.32 - 1.22 (m, 40H), 0.90 (td, J = 7.0, 1.8 Hz, 12H). LCMS (ESI): 853.2 [M+H]⁺.

### Example 17

### bis(2-octyldodecyl)5,5'-(((3R,4S)-1-(3-hydroxypropyl)pyrrolidine-3,4-diyl)bis(oxy))dipentanoate

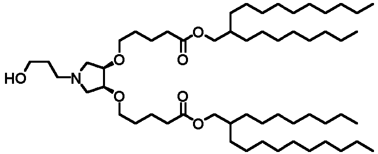

¹H NMR (600 MHz, CDCl₃) δ 3.99 (d, J = 5.8 Hz, 4H), 3.90 (t, J = 4.4 Hz, 2H), 3.85 - 3.73 (m, 2H), 3.57 (dt, J = 9.2, 6.3 Hz, 2H), 3.48 (dt, J = 9.2, 6.3 Hz, 2H), 3.27 (s, 2H), 2.85 (t, J = 5.9 Hz, 2H), 2.60 (dd, J = 9.8, 4.9 Hz, 2H), 2.36 (t, J = 7.4 Hz, 4H), 1.78 - 1.68 (m, 6H), 1.65 (dt, J = 9.4, 6.3 Hz, 6H), 1.29 (d, J = 7.9 Hz, 64H), 0.91 (t, J = 7.0 Hz, 12H). LCMS (ESI): 923.2 [M+H]⁺.

### Example 18

### bis(2-octyldodecyl)5,5'-(((3R,4S)-1-(5-hydroxypentyl)pyrrolidine-3,4-diyl)bis(oxy))dipentanoate

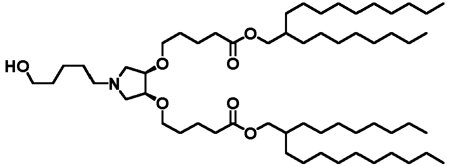

¹H NMR (600 MHz, CDCl₃) δ 3.99 (d, J = 5.8 Hz, 4H), 3.68 (td, J = 6.4, 1.8 Hz, 2H), 3.59 (d, J = 8.1 Hz, 2H), 3.50 (ddt, J = 11.8, 8.5, 3.9 Hz, 2H), 2.36 (t, J = 7.4 Hz, 4H), 1.72 (dq, J = 11.9, 7.3 Hz, 4H), 1.67 - 1.58 (m, 20H), 1.46 (d, J = 7.1 Hz, 2H), 1.36 - 1.23 (m, 62H), 0.91 (t, J = 6.9 Hz, 12H). LCMS (ESI): 951.2 [M+H]⁺.

### Example 19

### bis(2-octyldodecyl)5,5'-(((3R,4S)-1-(6-hydroxyhexyl)pyrrolidine-3,4-diyl)bis(oxy))dipentanoate

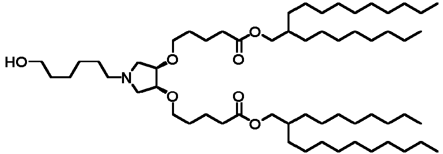

¹H NMR (600 MHz, CDCl₃) δ 3.98 (d, J = 5.8 Hz, 4H), 3.67 (t, J = 6.5 Hz, 2H), 3.60 (d, J = 8.4 Hz, 2H), 3.54 - 3.41 (m, 2H), 2.35 (t, J = 7.4 Hz, 4H), 1.71 (p, J = 7.2 Hz, 4H), 1.62 (d, J = 18.8 Hz, 22H), 1.40 (s, 4H), 1.29 (d, J = 8.6 Hz, 60H), 0.90 (t, J = 6.9 Hz, 12H). LCMS (ESI): 965.4 [M+H]⁺.

### Example 20

### bis(2-butyloctyl) 5,5'-(((3R,4S)-1-(3-hydroxypropyl)pyrrolidine-3,4-diyl)bis(oxy))dipentanoate

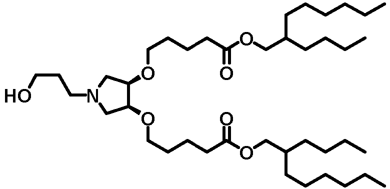

¹H NMR (600 MHz, CDCl₃) δ 3.98 (d, J = 5.8 Hz, 4H), 3.67 (t, J = 6.5 Hz, 2H), 3.60 (d, J = 8.4 Hz, 2H), 3.54 - 3.41 (m, 2H), 2.35 (t, J = 7.4 Hz, 4H), 1.71 (p, J = 7.2 Hz, 4H), 1.62 (d, J = 18.8 Hz, 22H), 1.40 (s, 4H), 1.29 (d, J = 8.6 Hz, 60H), 0.90 (t, J = 6.9 Hz, 12H); LCMS (ESI): 698.8 [M+H]⁺.

### Example 21

### bis(2-butyloctyl) 5,5'-(((3R,4S)-1-(5-hydroxypentyl)pyrrolidine-3,4-diyl)bis(oxy))dipentanoate

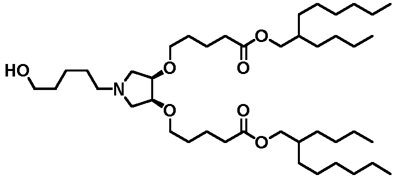

¹H NMR (600 MHz, CDCl₃) δ 3.99 (d, J = 5.8 Hz, 6H), 3.66 (t, J = 6.4 Hz, 2H), 3.59 (dt, J = 9.0, 6.4 Hz, 2H), 3.49 (dt, J = 9.1, 6.3 Hz, 2H), 2.35 (t, J = 7.4 Hz, 4H), 1.76 - 1.68 (m, 6H), 1.67 - 1.57 (m, 14H), 1.44 (p, J = 8.0 Hz, 2H), 1.35 - 1.25 (m, 32H), 0.91 (td, J = 6.7, 5.1 Hz, 12H); LCMS (ESI): 726.7 [M+H]⁺.

### Example 22

### bis(2-butyloctyl) 5,5'-(((3R,4S)-1-(6-hydroxyhexyl)pyrrolidine-3,4-diyl)bis(oxy))dipentanoate

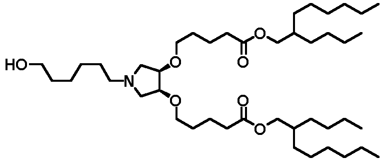

¹H NMR (600 MHz, CDCl₃) δ 3.99 (d, J = 5.8 Hz, 4H), 3.66 (t, J = 6.5 Hz, 2H), 3.59 (d, J = 7.9 Hz, 2H), 3.49 (dt, J = 9.2, 6.2 Hz, 2H), 2.35 (t, J = 7.4 Hz, 4H), 1.75 - 1.68 (m, 4H), 1.61 (tq, J = 21.1, 6.3 Hz, 20H), 1.39 (d, J = 14.3 Hz, 4H), 1.34 - 1.25 (m, 30H), 0.91 (td, J = 6.7, 5.1 Hz, 12H); LCMS (ESI): 740.9 [M+H]⁺.

### Lipid nanoparticles (LNPs) Preparation

The Firefly luciferase (FLuc) reporter mRNA-loaded lipid nanoparticles (LNPs) were formulated by NANOASSEMBLR^{®} IGNITE^{™}. Briefly, the lipid, cholesterol, 1,2-dimyristoyl-rac-glycero-3-methoxypolyethylene glycol-2000 (DMG-PEG2000), and 1,2-stearoyl-sn-glycerol-3-phosphocholine (DSPC) mixture was prepared in ethanolic solution at the % mole ratio of 50:38.5:10:1.5. Three units of FLuc mRNA was dissolved in 25 mM sodium acetate pH 5.0 buffer and one unit of lipid mixture were rapidly combined in the microfluidic mixing cartridge at an amine-to-phosphate (N/P) ratio constant at 6, and the instrument flow rate set at 12 mL/min. The mRNA-LNPs performed a 40-fold dilution of calcium and magnesium-free phosphate-buffered saline (PBS) to reach pH neutral. Then, LNPs were concentrated using Amicon^{®} Ultra-15 Centrifugal Filter Unit.

The mRNA (SEQ ID NO: 1) can be purchased from TRILINK BIOTECHNOLOGIES^{®}, LLC.

### Characterization

The mRNA-LNPs were characterized in terms of hydrodynamic size (z-average diameter), zeta potential, and polydispersity index by ZETASIZER^{™} PRO BLUE (MALVERN PANALYTICAL^{®}). A QUANT-IT^{™} RIBOGREEN assay kit (THERMOFISHER^{®}) was conducted to identify the mRNA concentration and encapsulation efficiency. In brief, mRNA-LNPs were diluted in equal volumes of Tris/EDTA (TE) buffer or 2% Triton X-100 in TE buffer. After that, a RIBOGREEN reagent was added to each sample and incubated at 37°C in absence of light for 15 min. The fluorescence intensity (Ex/Em 480/520 nm) was detected on a TECAN^{™} Microplate reader. The encapsulation efficiency (E.E) can be calculated by the formula: E.E (%) = (Total mRNA concentration - Unencapsulated mRNA concentration) / (Total mRNA concentration) × 100%

Furthermore, the acid dissociation constant (pKa) of mRNA-LNPs was determined using 6-(p-Toluidino)-2-naphthalenesulfonyl chloride (TNS) binding assay. Several pH range buffers (20 mM citrate buffers range from pH 3.0 to 5.5; 20 mM sodium phosphate buffers range from pH 6.0 to 8.0; 20 mM Tris buffers range from pH 8.5 to 10.0) were prepared. LNPs (0.5 mM total lipids) were mixed with the above buffer solution and TNS reagent to achieve a final concentration of TNS reagent being 6 µM in a total volume of 0.2 mL, and then incubated for ten minutes. The fluorescence intensity value was recorded by Infinite M200 PRO (Ex/Em 321/447 nm).

**Table 1 - Composition Analytics**

| **Compound** | **LNP pKa (TNS)** | **Encapsulation (%)** | **LNP size (nm)** | **PDI** | **LNP zeta potential (mV)** |
|---|---|---|---|---|---|
| **1** | 5.84 | 98.5 | 73.80 | 0.026 | -7.06 |
| **2** | 5.92 | 98.1 | 74.65 | 0.033 | -5.73 |
| **3** | 6.49 | 97.92 | 98.84 | 0.016 | 6.98 |
| **4** | 6.06 | 98.43 | 79.20 | 0.015 | 1.03 |
| **5** | 6.50 | 97.71 | 86.05 | 0.010 | 4.37 |
| **6** | 6.55 | 96.87 | 78.86 | 0.030 | 8.21 |
| **7** | 6.53 | 97.22 | 87.10 | 0.025 | 2.41 |
| **8** | 6.59 | 97.75 | 86.87 | 0.035 | 2.60 |
| **9** | 6.97 | 94.43 | 102.00 | 0.066 | 9.61 |
| **10** | 6.80 | 96.63 | 82.64 | 0.023 | -0.46 |
| **11** | 6.58 | 97.58 | 80.71 | 0.014 | 10.95 |
| **12** | 6.73 | 96.99 | 78.35 | 0.039 | 7.83 |
| **13** | 6.70 | 97.39 | 81.29 | 0.046 | 12.45 |
| **14** | 7.00 | 96.90 | 82.45 | 0.050 | 14.09 |
| **15** | 7.13 | 96.75 | 81.53 | 0.051 | 10.29 |
| **16** | 6.96 | 96.34 | 84.99 | 0.045 | 10.71 |
| **17** | 6.47 | 98.16 | 95.10 | 0.03 | 8.00 |
| **18** | 6.54 | 97.57 | 86.61 | 0.03 | 9.52 |
| **19** | 6.57 | 97.31 | 79.74 | 0.05 | 6.25 |
| **20** | 8.55 | 97.36 | 88.33 | 0.06 | 15.00 |
| **21** | 7.28 | 98.87 | 93.39 | 0.0532 | 14.91 |
| **22** | 6.96 | 98.18 | 148.20 | 0.05 | 10.78 |

### Transfection efficiency and cytotoxicity in HEPG2 cell lines

To investigate the potential and cytotoxicity of mRNA-LNP made from novel lipids, FLuc mRNA (TRILINK^{®}) was encapsulated into LNPs and transfected to the cell line. HEPG2 cells were maintained at 37°C under 5% CO₂ in a DMEM medium with 10% fetal bovine serum. Cells were seeded at 4×10⁴ cells/well and treated with FLuc mRNA-LNPs for 18 hours while cell confluency reach 70%. To explore transfection efficiency, HEPG2 cells were treated with D-luciferin and mixed thoroughly for another incubation of 5 min at 37°C. The luminescence intensity was recorded using M200 Microplate Reader (TECAN^{®}). Cell viability was evaluated by Cell Counting Kit-8 (SIGMA-ALDRICH^{®}) and the absorbance was measured at 450 nm. The results are shown in Table 2.

**Table 2 - Transfection efficiency and cytotoxicity**

| **Compound** | **LNP pKa (TNS)** | **Encapsulation (%)** | **LNP size (nm)** | **PDI** | **LNP zeta potential (mV)** | **Transfection efficiency (relative to SM-102, %)** | **Cytotoxicity in HEPG2^{#}** |
|---|---|---|---|---|---|---|---|
| **11** | 6.58 | 97.58 | 80.71 | 0.014 | 10.95 | 100% | - |
| **14** | 7.00 | 96.90 | 82.45 | 0.050 | 14.09 | 105% | - |

The toxicity of compounds 11 and 14 is less than 10%.

While the present disclosure has been described and illustrated with reference to specific embodiments thereof, these descriptions and illustrations are not limiting. It should be understood by those skilled in the art that various changes may be made and equivalents may be substituted without departing from the true spirit and scope of the present disclosure as defined by the appended claims. The illustrations may not be necessarily drawn to scale. There may be distinctions between the artistic renditions in the present disclosure and the actual apparatus due to manufacturing processes and tolerances. There may be other embodiments of the present disclosure which are not specifically illustrated. The specification and drawings are to be regarded as illustrative rather than restrictive. Modifications may be made to adapt a particular situation, material, composition of matter, method, or process to the objective, spirit and scope of the present disclosure. All such modifications are intended to be within the scope of the claims appended hereto. While the methods disclosed herein have been described with reference to particular operations performed in a particular order, it will be understood that these operations may be combined, sub-divided, or re-ordered to form an equivalent method without departing from the teachings of the present disclosure. Accordingly, unless specifically indicated herein, the order and grouping of the operations are not limitations of the present disclosure.

## Claims

1. A lipid of Formula (I), or pharmaceutically acceptable salts, solvates, hydrates, polymorphs, tautomers, stereoisomers, or isotopically enriched derivatives thereof, 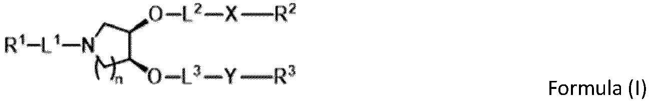 wherein:
n is 1 or 2;
R¹ is amino, monoalkylamino, dialkylamino, hydroxy, alkoxy, carbamoyl, monoalkylcarbamoyl, dialkylcarbamoyl, pyrrolidinyl, piperidyl or morpholinyl;
R² and R³ are each independently an alkyl, alkenyl or alkynyl having 5 to 30 carbon atoms;
L¹ is an alkyl having 2 to 6 carbon atoms;
L² and L³ are each independently an alkyl having 2 to 9 carbon atoms; and
X and Y are each independently -C(O)-O- or -O-C(O)-.

2. The lipid or pharmaceutically acceptable salts, solvates, hydrates, polymorphs, tautomers, stereoisomers, or isotopically enriched derivatives thereof of claim 1, wherein:
n is 1;
R¹ is hydroxy;
R² and R³ are each independently -CH((CH₂)ₚCH₃)((CH₂)_{q}CH₃) or -(CH₂)ₘCH((CH₂)ₚCH₃)((CH₂)_{q}CH₃), wherein m is an integer from 1 to 3, and p and q are each independently an integer from 1 to 12;
L¹ is an alkyl having 2 to 6 carbon atoms;
L² and L³ are each independently an alkyl having 2 to 6 carbons atoms; and
X and Y are each independently -C(O)-O- or -O-C(O)-; or
n is 1;
R¹ is hydroxy;
R² and R³ are each independently -CH((CH₂)ₚCH₃)((CH₂)_{q}CH₃) or -CH₂CH((CH₂)ₚCH₃)((CH₂)_{q}CH₃), wherein p and q are each independently an integer from 3 to 9;
L¹ is an alkyl having 2 to 6 carbon atoms;
L² and L³ are each independently an alkyl having 2 to 6 carbons atoms; and
X and Y are each independently -C(O)-O- or -O-C(O)-.

3. The lipid or pharmaceutically acceptable salts, solvates, hydrates, polymorphs, tautomers, stereoisomers, or isotopically enriched derivatives thereof of claim 1 or 2, which lipid is selected from the group consisting of:
di(heptadecan-9-yl)3,3'-(((3R,4S)-1-(2-hydroxyethyl)pyrrolidine-3,4-diyl)bis(oxy))dipropionate, di(heptadecan-9-yl)3,3'-((3R,4S)-1-(3-hydroxypropyl)pyrrolidine-3,4-diyl)bis(oxy))propionate, di(heptadecan-9-yl)5,5'-(((3S,4R)-1-(3-hydroxypropyl)pyrrolidine-3,4-diyl)bis(oxy))dipentanoate, di(heptadecan-9-yl)5,5'-(((3S,4R)-1-(2-hydroxyethyl)pyrrolidine-3,4-diyl)bis(oxy))dipentanoate, (((3S,4R)-1-(3-hydroxypropyl)pyrrolidine-3,4-diyl)bis(oxy))bis(pentane-5,1-diyl)bis(2-hexyldecanoate),
(((3S,4R)-1-(2-hydroxyethyl)pyrrolidine-3,4-diyl)bis(oxy))bis(pentane-5,1-diyl)bis(2-hexyldecanoate), di(heptadecan-9-yl)3,3'-(((3R,4S)-1-(4-hydroxybutyl)pyrrolidine-3,4-diyl)bis(oxy))dipropionate, (((3R,4S)-1-(3-hydroxypropyl)pyrrolidine-3,4-diyl)bis(oxy))bis(butane-4,1-diyl)bis(2-hexyldecanoate), (((3S,4R)-1-(5-hydroxypentyl)pyrrolidine-3,4-diyl)bis(oxy))bis(pentane-5,1-diyl)bis(2-hexyldecanoate),
(((3S,4R)-1-(6-hydroxyhexyl)pyrrolidine-3,4-diyl)bis(oxy))bis(pentane-5,1-diyl)bis(2-hexyldecanoate),
di(heptadecan-9-yl)6,6'-(((3R,4S)-1-(3-hydroxypropyl)pyrrolidine-3,4-diyl)bis(oxy))dihexanoate, di(heptadecan-9-yl)6,6'-(((3R,4S)-1-(5-hydroxypentyl)pyrrolidine-3,4-diyl)bis(oxy))dihexanoate, di(heptadecan-9-yl)6,6'-(((3R,4S)-1-(4-hydroxybutyl)pyrrolidine-3,4-diyl)bis(oxy))dihexanoate, (((3S,4R)-1-(3-hydroxypropyl)pyrrolidine-3,4-diyl)bis(oxy))bis(hexane-6,1-diyl)bis(hexyldecanoate), (((3S,4R)-1-(5-hydroxypentyl)pyrrolidine-3,4-diyl)bis(oxy))bis(hexane-6,1-diyl)bis(2-hexyldecanoate),
(((3S,4R)-1-(4-hydroxybutyl)pyrrolidine-3,4-diyl)bis(oxy))bis(hexane-6,1-diyl)bis(2-hexyldecanoate), bis(2-octyldodecyl)5,5'-(((3R,4S)-1-(3-hydroxypropyl)pyrrolidine-3,4-diyl)bis(oxy))dipentanoate, bis(2-octyldodecyl)5,5'-(((3R,4S)-1-(5-hydroxypentyl)pyrrolidine-3,4-diyl)bis(oxy))dipentanoate, bis(2-octyldodecyl)5,5'-(((3R,4S)-1-(6-hydroxyhexyl)pyrrolidine-3,4-diyl)bis(oxy))dipentanoate, bis(2-butyloctyl)5,5'-(((3R,4S)-1-(3-hydroxypropyl)pyrrolidine-3,4-diyl)bis(oxy))dipentanoate, bis(2-butyloctyl)5,5'-(((3R,4S)-1-(5-hydroxypentyl)pyrrolidine-3,4-diyl)bis(oxy))dipentanoate, and bis(2-butyloctyl)5,5'-(((3R,4S)-1-(6-hydroxyhexyl)pyrrolidine-3,4-diyl)bis(oxy))dipentanoate.

4. The lipid or pharmaceutically acceptable salts, solvates, hydrates, polymorphs, tautomers, stereoisomers, or isotopically enriched derivatives thereof of any one of claims 1 to 3, wherein the pKₐ of a protonated form of the lipid is about 5.0 to about 9.0.

5. An active agent delivery vehicle comprising the lipid or pharmaceutically acceptable salts, solvates, hydrates, polymorphs, tautomers, stereoisomers, or isotopically enriched derivatives thereof of any one of claims 1 to 4.

6. The active agent delivery vehicle of claim 5, which is selected from the group consisting of liposomes, single emulsions, micelles, nanoemulsions, lipid nanoparticles (LNPs), nanostructured lipid carriers (NLC), and self-emulsifying drug delivery system (SEDDS).

7. The active agent delivery vehicle of claim 5 or 6, further comprising one or more lipids selected from the group consisting of neutral lipids, steroid lipids, and PEGylated lipids.

8. The active agent delivery vehicle of claim 7, wherein the neutral lipids are selected from the group consisting of 1,2-dilinoleoyl-sn-glycero-3-phosphocholines (DLPC), 1,2-dimyristoleoyl-sn-glycero-3-phosphocholines (DMPC), 1,2-dioleoyl-sn-glycero-3-phosphocholines (DOPC), 1,2-dipalmitoyl-sn-glycero-3-phosphocholines (DPPC), 1,2-distearoyl-sn-glycero-3-phosphatidylcholines (DSPC), 1,2-diundecanoyl-sn-glycero-3-phosphatidylcholines (DUPC), 1-plamitoyl-2-oleoyl-sn-glycero-3-phosphocholines (POPC), 1,2-di-O-octadecenyl-sn-glycero-3-phosphatidylcholines (18:0 Diether PC), 1-oleoyl-2-cholesterylhemisuccinyl-sn-glycero-3-phosphocholines (OChemsPC), 1-O-hexadecyl-sn-glycero-3-phosphatidylcholines (C16 Lyso PC), 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines (DOPE), 1,2-diphytanyl-sn-glycero-3-phosphoethanolamines (ME 16.0 PE), 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3-phosphoethanolamines, 1,2-dioleoyl-sn-glycero-3-phospho-rac-(1-glycerol) sodium salts (DOPG), dioleoyl phosphatidylserines (DOPS), dipalmitoylphosphatidylglycerols (DPPG), palmitoyloleoyl phosphatidylethanolamines (POPE), distearoyl phosphatidylethanolamines (DSPE), dipalmitoyl phosphatidylethanolamines (DPPE), dimyristoleoyl phosphoethanolamines (DMPE), 1-stearoyl-2-oleoyl-stearoylethanolamines (SOPE), 1-stearoyl-2-oleoyl-phosphatidylcholines (SOPC), sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, and lysophosphatidylethanolamines (LPE), and combinations thereof.

9. The active agent delivery vehicle of claim 7, wherein the steroid lipids are selected from the group consisting of cholesterols, coprostanols, sitosterols, ergosterols, campesterols, stigmasterols, brassicasterol tomatidines, ursolic acids, and α-tocopherols, and combinations thereof.

10. The active agent delivery vehicle of claim 7, wherein the PEGylated lipids are selected from the group consisting of 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), and PEG-dialkyloxypropyl (PEG-DAA), and combinations thereof.

11. A composition comprising the active agent delivery vehicle of any one of claims 5 to 10 and an active agent and optionally one or more pharmaceutically acceptable excipients or adjuvants.

12. The composition of claim 11, wherein the active agent includes a nucleic acid, and the nucleic acid is selected from the group consisting of antisense oligonucleotide, plasmid, interfering nucleic acid, aptamer, microRNA (miRNA), Dicer-substrate RNA (dsRNA), small hairpin RNA (shRNA), mRNA (messenger RNA), guide RNA (gRNA), miRNA inhibitor (antagomir), and ribozyme, and combinations thereof.

13. The composition of claim 11 or 12 for use in treating or preventing a disease or disorder.

14. A method for introducing a nucleic acid into a target cell or transfecting the target cell with the nucleic acid, comprising bringing the composition of claim 11 or 12 into contact with the target cell.

15. A method of modulating the expression of a target gene in a cell, comprising providing to the cell the composition of claim 11 or 12.
